(19) **Europäisches Patentamt / European Patent Office / Office européen des brevets**

(11) **EP 1 679 091 B1**

(12) **EUROPEAN PATENT SPECIFICATION**

(45) Date of publication and mention
of the grant of the patent:
**26.01.2011 Bulletin 2011/04**

(51) Int Cl.:
**A61M 5/145** (2006.01)

(21) Application number: **06005799.9**

(22) Date of filing: **14.12.2000**

(54) **Syringe pump**

Spritzenpumpe

Pompe seringue

(84) Designated Contracting States:
**AT BE CH CY DE DK ES FI FR GB GR IE IT LI LU
MC NL PT SE TR**

(30) Priority: **24.12.1999 JP 36702599**

(43) Date of publication of application:
**12.07.2006 Bulletin 2006/28**

(62) Document number(s) of the earlier application(s) in
accordance with Art. 76 EPC:
**00311194.5 / 1 110 569**

(73) Proprietor: **TERUMO KABUSHIKI KAISHA
Tokyo 151-0072 (JP)**

(72) Inventors:
• **Watanabe, Takashi
Fujinomiya-shi, Shizuoka-ken (JP)**
• **Nakada, Narukuni
Fujinomiya-shi, Shizuoka-ken (JP)**

(74) Representative: **Paget, Hugh Charles Edward et al
Mewburn Ellis LLP
33 Gutter Lane
London
EC2V 8AS (GB)**

(56) References cited:
EP-A- 0 314 880    EP-A- 0 514 907
EP-A- 0 916 353    WO-A-94/08647
WO-A-96/25963    JP-A- 09 122 237
US-A- 5 425 716

**Description**

FIELD OF THE INVENTION

The present invention relates to a syringe pump in which one of various types of different syringes is detachably held in an immobile state, the thumb rest (or piston rod) of the syringe is set in the immobile state witch respect to a slider means, and the slider means is driven by a predetermined amount, thereby feeding the content of the syringe accurately, and to a syringe pump in which most of its operation is automated

BACKGROUND OF THE INVENTION

A syringe pump mainly aims at supply of nutrition, blood transfusion, and injection of a liquid drug such as chemotherapeutics, narcotic, and the like at high precision in an intensive care unit (ICU), coronary care unit (CCU), or neonatal intensive care unit (NICU), and is often used since liquid flow rate control and the like can be controlled better than in pumps of other types.

The syringe pump is used in the following manner. A syringe containing a liquid drug is set in a syringe pump in an immobile state by using the clamp of the syringe pump, and the thumb rest of the syringe is set on the slider. The slider is then driven to feed the content in the syringe accurately upon piston motion of the thumb rest (or the piston rod). The syringe pump is often used since it has a simple structure and comparatively high precision.

In liquid drug injection performed in this manner, if the syringe is used with the flange of its thumb rest not being fixed to the slider, the liquid drug is not fed from the syringe. When the syringe is connected to a closed circuit, a negative pressure that moves the thumb rest to the injection side is generated. In this case, if the thumb rest is not set in the immobile state, a liquid drug in an amount exceeding the preset amount is sometimes injected.

Many syringe pumps have a function of detecting whether the flange of the thumb rest of the syringe is set on the slider portion, which fixes the flange of the thumb rest (or the piston rod) for the syringe, in the immobile state, or is disengaged from the slider portion, and enables injection only when the flange of the thumb rest of the syringe is set on the slider portion in the immobile state, and prohibits injection when it is not.

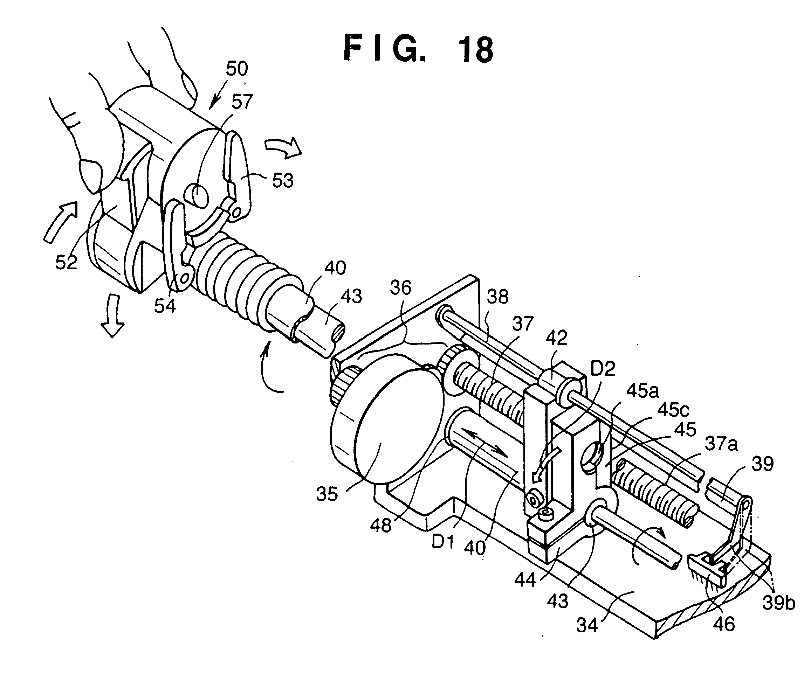

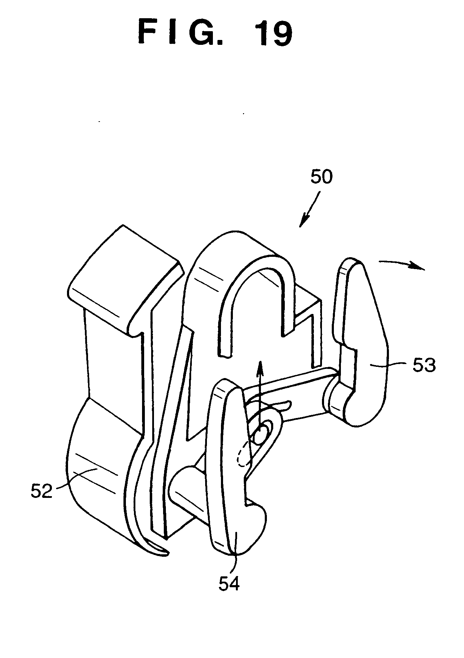

A syringe pump having the above function is disclosed in, e.g., Japanese Patent Laid-Open No. 9-122237. The arrangement of this syringe pump will be described with reference to Fig. 18 as the perspective view of the outer appearance of a slider mechanism and Fig. 19 as the perspective view of the outer appearance for explaining its operation. When the main body of syringe (not shown) is set on the main body of the syringe pump in the immobile state, the flange (not shown) of the thumb rest of the syringe is set on a slider assembly 50 driven in directions of arrows in Fig. 18. The slider assembly 50 is moved reciprocally, and simultaneously connected and fixed to a pipe shaft serving as a hollow shaft with respect to a slider feed mechanism, and to the end of an inner clutch shaft serving as a clutch shaft. When a clutch lever 52 of the slider assembly 50 is manually operated, the flange of the thumb rest can be mounted and removed easily.

In the above arrangement, as shown in Fig. 19, when the user grips the clutch lever 52 in order to set the syringe, a pair of right and left hooks 53 and 54 open in synchronism with each other, so that one of the flanges with different sizes of thumb rests can be placed.

A base 34 serving as the base portion is integrally formed with right and left wall surfaces (one of them is not shown), so that a lead screw 37 with one end to which the gear of a gear train 36 is fixed, a clutch shaft 43, and a guide shaft 38 are supported in a space portion sandwiched by the right and left wall surfaces. The lead screw 37 is pivotally supported by a bearing.

A pipe shaft 40 movable through a guide bush 48 in the directions of arrows D1 is coaxially, slidably guided on the clutch shaft 43. The guide shaft 38 is fixed to the base 34 in the immobile state. A block 41 for fixing a polyacetal resin rotation-preventive resin bush 42 in which the guide shaft 38 is inserted is fixed to the end of the pipe shaft 40. A nut holder 44 for fixing a half nut member 45, always biased in the direction of a blank arrow D2 and having a tooth portion 45a always meshing with a tooth portion 37a of the lead screw 37, is fixed to the clutch shaft 43. The nut holder 44 is disengaged and set in the free state only when the clutch shaft 43 is pivoted in a direction opposite to the arrow D2, so that it can freely move in the directions of arrows D1.

A clutch sensor plate 39 serving as the dog means of a sensor is pivotally, axially supported by the guide shaft 38. When the user grips the clutch lever 52 of the slider assembly 50, the half nut member 45 is rotated through the pipe shaft 40, and consequently the ridge portion 37a of the lead screw 37 and the ridge portion 45a of the half nut member 45 are disengaged from each other. When the half nut member 45 is rotated in this manner, the clutch sensor plate 39, biased by a biasing means (not shown) such as a coil spring, a leaf spring, and the like so its slide surface 39c always abuts against a slide surface 45c of the half nut member 45, is rotated simultaneously. When the clutch sensor plate 39 rotates, light is transmitted to the sensor serving as a photosensor (transmission type) 46 fixed inside the upper cover and normally light-blocked by a lever portion 39b at one end of the clutch sensor plate 39.

When this light transmission is detected, an alarm indicating that the syringe is not mounted is displayed. When a motor 35 is driven in order to move the flange of the thumb rest, the lead screw 37 is rotationally driven, and the nut holder 44 fixing the half nut member 45 which meshes with the lead screw 37 is moved. Hence, the pipe shaft 40 is moved to move the slider assembly 50. At this time, the lever portion 39b at one end of the clutch sensor plate 39 is set in the state indicated by a solid line in Fig. 18 where it blocks light to the photosensor (transmission type) 46. A contact pin 57 biased such that its one end always projects by the operation of a torsion spring is provided at a portion on a side surface of the base of the slider assembly 50. The starting state can be set only when the flange of the thumb rest abuts against the contact pin 57 to move it inward.

In a syringe disengagement detection apparatus of European Patent No. 514907B1, a technique is disclosed which enables detection of disengagement of the syringe during operation by optically detecting the motion of a component corresponding to the contact pin provided to the slider assembly 50 described above. US patent 5,425,716 discloses a syringe pump with diameter detecting means, reading means, storage means for manufacturer settings and discriminating means and control means said document does not disclose avoiding balises when occlusion is detected occlusion is detected.

SUMMARY OF THE INVENTION

The present invention provides a syringe pump according to claim 1.

The present invention provides a syringe pump in which occlusion detection can be performed more accurately for various types of injection patterns (program input patterns) and various types of syringes, and drive control of the syringe can be performed without causing bolus, even when occlusion is detected.

The present invention provides a syringe pump which can arbitrarily select a occlusion pressure for various types of syringes in accordance with syringe capacities (syringe diameters) and syringe manufacturers or for a syringe containing a predetermined liquid drug, so that it can detect an occlusion pressure precisely.

BRIEF DESCRIPTION OF THE DRAWINGS

Fig. 1 is a partially cutaway perspective view of the outer appearance of a slider mechanism to show a base 34 serving as a base portion;
Fig. 2 is a perspective view of the outer appearance of a slider assembly 50 from which a syringe pump cover is removed;
Fig. 3 is a sectional view taken along the line of arrows Y - Y of Fig. 1;
Fig. 4 is a side view of Fig. 2;
Fig. 5 is a sectional view taken along the line of arrows X - X of Fig. 4 for explaining the operation of a contact pin 1;
Fig. 6 is a sectional view taken along the line of arrows X - X of Fig. 1 for showing a meshing state during operation;
Fig. 7 is a sectional view taken along the line of arrows X - X of Fig. 1 for showing an inoperative state;
Fig. 8 is a table showing operation specifications;
Fig. 9 is a perspective view of the outer appearance of the syringe pump;
Fig. 10 is a plan view of an operation panel;
Fig. 11 is a block diagram of the syringe pump;
Figs. 12 and 13 are flow charts for explaining the operation of the syringe pump of the present invention;
Figs. 14 and 15 are flow charts for explaining the operation of a comparative syringe pump;
Figs. 16 and 17 are flow charts for explaining the operation of a comparative syringe pump;
Fig. 18 is a perspective view of the outer appearance of a conventional slider mechanism; and
Fig. 19 is a perspective view of the outer appearance of a conventional slider assembly 50.

DETAILED DESCRIPTION

The present invention will be described with reference to the accompanying drawings.

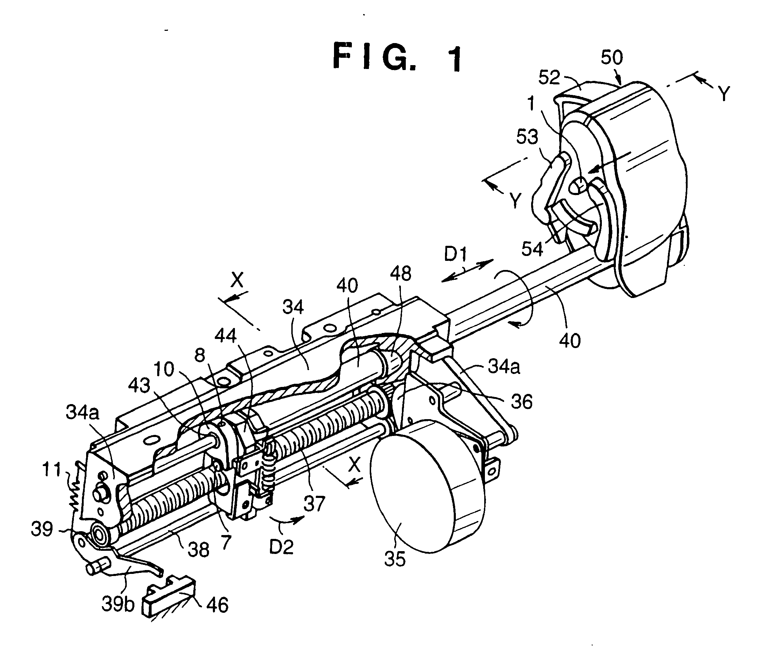

Fig. 1 is a partially cutaway perspective view of the outer appearance of a slider mechanism to show a base 34 serving as a base portion.

Referring to Fig. 1, when the main body of syringe (not shown) is set on the main body of the syringe pump in the immobile state, the thumb rest is set on a slider assembly 50 driven in directions of arrows in Fig. 1.

The slider assembly 50 meshes with a lead screw as will be described later so that it is driven to move. Simultaneously, the slider assembly 50 is connected and fixed to a pipe shaft 40 serving as a hollow shaft with respect to a slider feed mechanism, and to the end of a clutch shaft 43. When a clutch lever 52 of the slider assembly 50 is manually operated, hooks 53 and 54 are opened and closed, so that the flange (not shown) of the thumb rest can be mounted and removed easily.

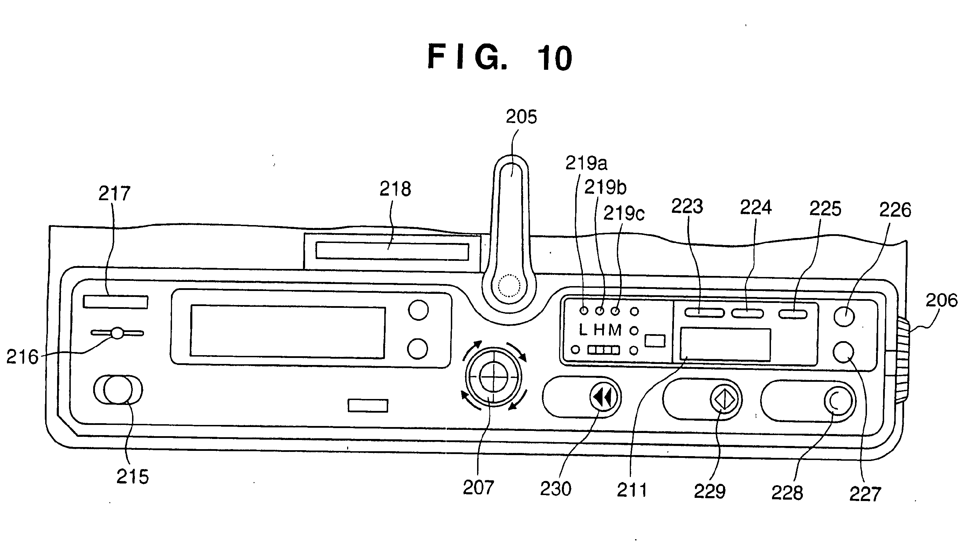

In the above arrangement, when the user grips the clutch lever 52 in order to set the syringe, the pair of left and right hooks 53 and 54 are opened in synchronism with each other, as has been described with reference to Fig. 10 as well, so that one of the flanges with different sizes of thumb rests can be held and placed on the slider assembly 50 in the substantially immobile state.

The base 34 serving as the base portion is integrally formed with right and left wall surfaces 34a by aluminum die casting, so that a lead screw 37 with one end to which the gear of a gear train 36 is fixed, the clutch shaft 43, and a guide shaft 38 also serving as the pivot fulcrum of a dog means are supported in a space portion sandwiched by the right and left wall surfaces 34a. The lead screw 37 is pivotally supported by a bearing.

The pipe shaft 40 movable through a guide bush 48 in directions of arrows D1 is coaxially, slidably guided on the clutch shaft 43. The guide shaft 38 is fixed to the base 34 in the immobile state. A block for fixing a polyacetal resin rotation-preventive bush in which the guide shaft 38 is inserted is fixed to the end of the pipe shaft 40.

A nut holder 44 for fixing a half nut member 45, always biased in the direction of an arrow D2 and having a tooth portion 45a always meshing with a tooth portion 37a of the lead screw 37, is provided to the clutch shaft 43, as will be described later. The nut holder 44 is disengaged and set in the free state only when the clutch shaft 43 is pivoted in a direction opposite to the arrow D2, so that it can freely move in the directions of arrows D1 to cover substantially the entire length of the syringe.

A clutch sensor plate 39 serving as a dog means is pivotally, axially supported by the guide shaft 38. When the user grips the clutch lever 52 of the slider assembly 50, the half nut member 45 is rotated through the pipe shaft 40, as will be described later, and consequently the ridge portion 37a of the lead screw 37 and the ridge portion 45a of the half nut member 45 are disengaged from each other.

When the half nut member 45 is rotated in this manner, the clutch sensor plate 39, biased by a biasing means (not shown) so its slide surface 39c always abuts against a slide surface 45c of the half nut member 45, is rotated simultaneously. When the clutch sensor plate 39 is rotated, light is transmitted to a sensor serving as a photosensor (transmission type) 46 fixed inside the upper cover and normally light-blocked by a lever portion 39b at one end of the clutch sensor plate 39.

When this light transmission is detected, an alarm indicating that the syringe is not mounted is displayed.

When a motor 35 is driven in order to move the flange of the thumb rest, the lead screw 37 is rotationally driven, and the nut holder 44 fixing the half nut member 45 meshing with the lead screw 37 is moved. Hence, the pipe shaft 40 is moved to move the slider assembly 50. At this time, the lever portion 39b at one end of the clutch sensor plate 39 blocks light to the photosensor (transmission type) 46. A contact pin (contact member) 1 biased by a torsion spring such that its one end always projects is provided at a portion on a side surface of the base of the slider assembly 50. The starting state by the user described above can be set only when the flange of the thumb rest abuts against the contact pin 1 to move it inward.

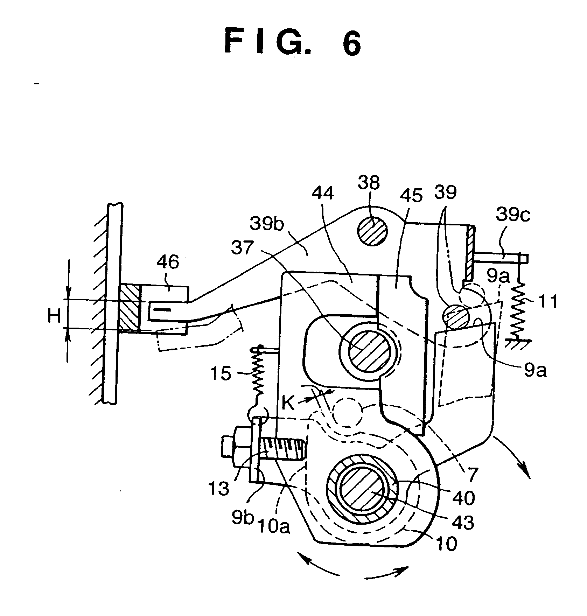

A further explanation will be made with reference to Fig. 6, which is a sectional view taken along the line of arrows X - X of Fig. 1. A power transmission mechanism is comprised of an operation member 10 fixed to one end of the clutch shaft 43 and having a projection 10a, the half nut member 44 pivotally provided to the clutch shaft 43, a stud 7 (indicated by a broken line) fixed to the side surface of a half nut member 44 to be away from the projection 10a of the operation member 10 by a predetermined gap K, and an intermediate member 9 obtained by integrally forming an abutting portion 9a, serving as a dog means pivotally provided to the base 34 and having one end abutting against the shaft 39, a hole portion pivotally supported by the clutch shaft 43, and a portion 9b provided with a spring 15 serving as a biasing means to bias the operation member 10 to separate from the stud 7 by a distance corresponding to the clearance K described above.

In the above arrangement, referring to Fig. 6, the dog portion 39b of the dog means is located at the position indicated by a solid line due to the operation of a tensile spring 11 serving as a biasing means. In the operative state wherein the lead screw 37 meshes with the half nut member 45, the dog portion 39b blocks light to the optical photosensor 46.

As will be described later, when the abutting state of the flange of the thumb rest is canceled, the contact pin 1 projects, and the clutch shaft 43 initially pivots only slightly, then the abutting surface 10a of the operation member 10 pivots in the direction of arrows from the abutting state with respect to a screw 13 against the tensile force of the spring 15, and pivots by a distance corresponding to the clearance K. As a result, the abutting portion 9a of the intermediate member 9 pivots to the position indicated by a broken line to move the shaft 39 of the dog means to the position indicated by a broken line in Fig. 6. Consequently, the dog portion 39b moves for a distance H to cancel the light-blocking state of the photosensor 46.

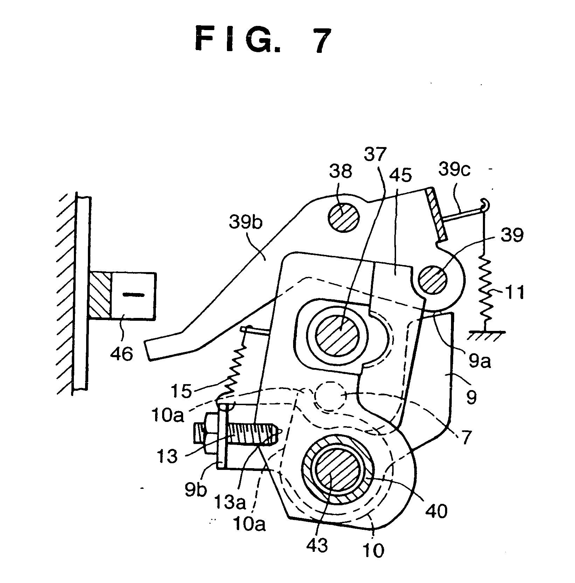

In Fig. 7, when the user grips the clutch lever 52 to further pivot the operation member 10 to the position indicated by a broken line, the operation member 10 presses the stud 7 to completely disengage the half nut member 45.

As the flange of the thumb rest is disengaged, the contact pin 1 projects and the clutch shaft 43 initially pivots only slightly. This pivot state can be detected in the above manner.

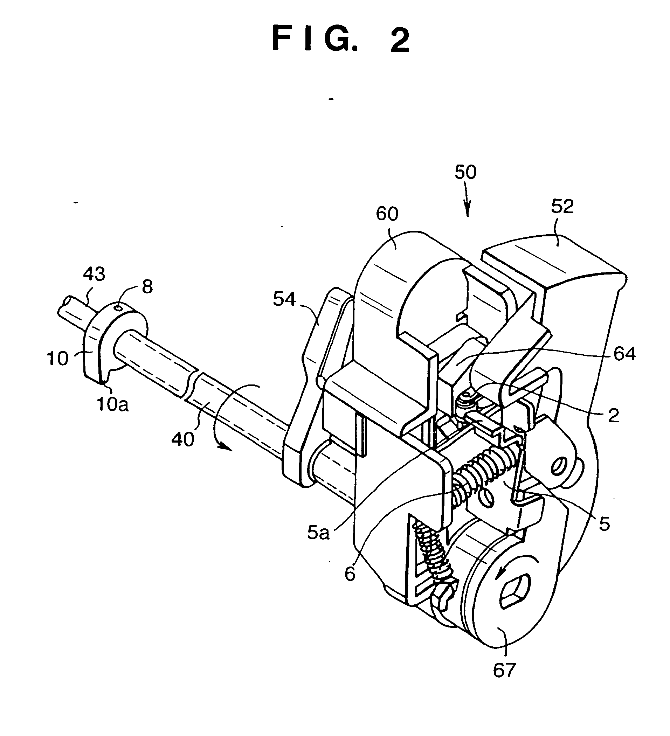

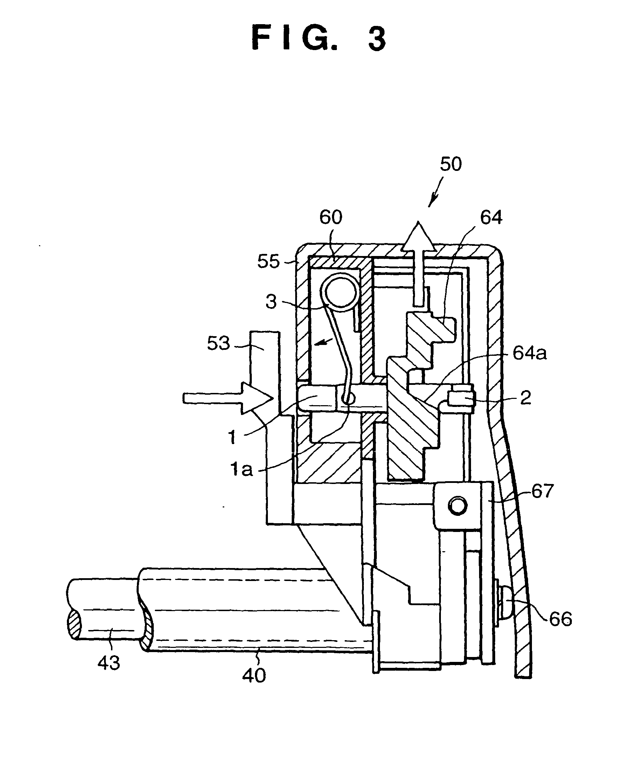

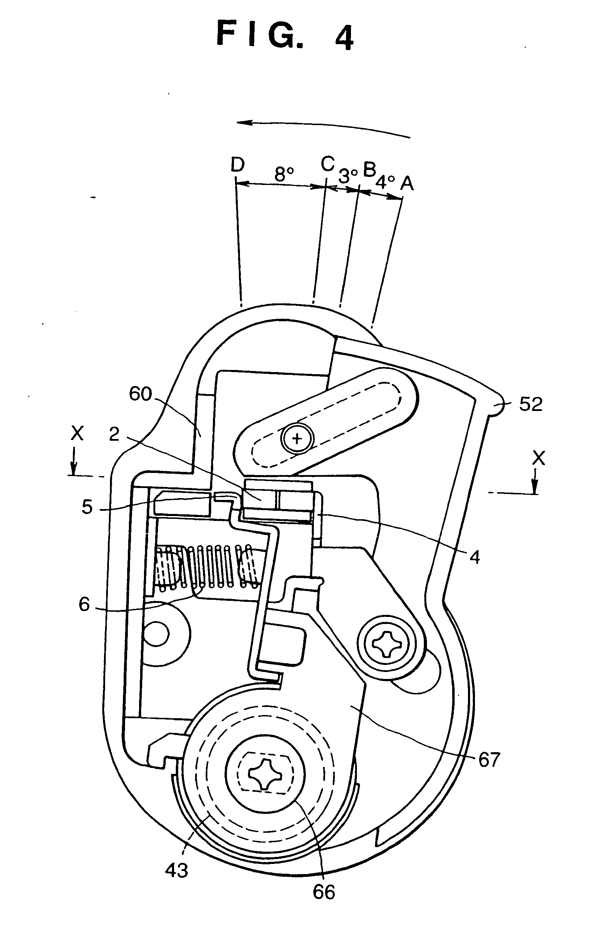

Fig. 2 is a perspective view of the outer appearance of the slider assembly 50 from which a cover is removed, Fig. 3 is a sectional view taken along the line of arrows Y - Y of Fig. 1, and Fig. 4 is a side view. In Figs. 2 to 4, portions that are already described are denoted by the same reference numerals, and a detailed description thereof will be omitted. A tensile spring (not shown) extends above a slider base 60 between the hooks of the clutch lever 52, and biases the left and right hooks 53 and 54 in a normally occlusion state.

The end of the clutch shaft 43 has a locking portion formed by parallel cutting. A plate 67 made of a thick stainless steel plate is fixed to this locking portion with a screw 66. Hence, as the clutch lever 52 pivots, an adjusting bolt fixed to the plate 67 abuts against the end of the clutch shaft 43. The clutch lever 52 is supported to be pivotal about the pipe shaft 40 described above as the center.

A plate cam component 64 has a slant surface 64a, as shown in Fig. 3, and the contact pin 1 is provided to be movable along the slant surface 64a. When the clutch lever 52 is moved to move the plate cam component 64 in a direction of arrow, the left and right hooks 53 and 54 open.

The contact pin 1 extends through the opening of a cover 55 and slides on the plate cam component 64.

More specifically, upon operation of the clutch lever 52, when the hooks 53 and 54 are opened, the motion of the clutch lever 52 is transmitted to the clutch shaft 43 through the plate 67, and the slider assembly 50 is disengaged from a slider feed mechanism 33. Thus, the slider assembly 50 can move in the longitudinal direction.

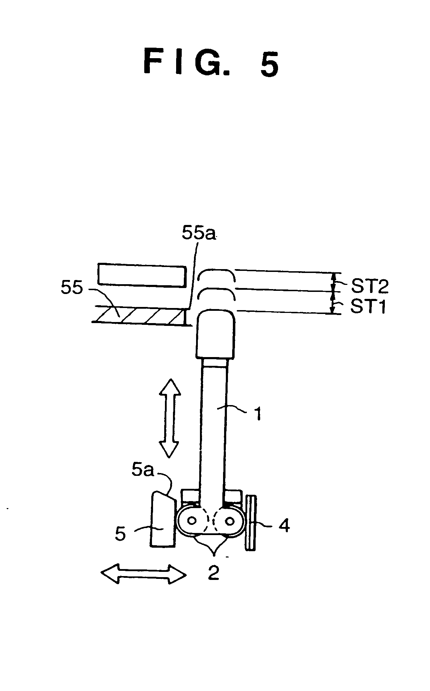

The contact pin 1 axially supports a pair of rollers 2 as shown in Fig. 5 which is the sectional view taken along the line of arrows X - X of Fig. 4. The rollers 2 are sandwiched between a wall surface 4 and a contact pin guide 5. For this purpose, a slant surface 5a and coil spring 6 are provided to the contact pin guide 5. When the contact pin 1 is moved in the direction of a double-headed arrow (vertical directions in Fig. 5), the contact pin guide 5 moves in the direction of double-headed arrow (to the right-to-left direction in Fig. 5).

Since the guide member 5 is fixed to the plate 67, as shown in Fig. 4, it pivots the clutch shaft 43 among positions A, B, C, and D. In the above arrangement, when the flange of the thumb rest of the syringe is not set on the slider assembly 50, the contact pin 1 projects as it is biased by a torsion spring 3. When the user grips the clutch lever 52 to set the flange of the thumb rest, the plate cam component 64 moves upward along a groove formed in the clutch lever 52, and the contact pin 1 is withdrawn inward along the slant surface 64a of the plate cam component 64. Since the plate cam component 64 moves upward, the left and right hooks 53 and 54 are also opened.

Simultaneously with this motion, upon operation of the lever 52, the clutch shaft 43 is pivoted to disengage the half nut member 45 from the lead screw 37, so that the slider assembly 50 can move.

When the user grips thumb rest of the syringe while gripping the clutch lever 52 and then releases the clutch lever 52, the contact pin 1 interferes with the return movement of the contact pin guide 5 as it is pressed by the thumb rest of the syringe. When the thumb rest is not set, the contact pin 1 moves in accordance with the return movement of the clutch lever 52, so the contact pin guide 5 also returns.

The motion of the contact pin guide 5 is transmitted to the operation member 10 through the clutch shaft 43, as described above, and is detected by the photosensor 46, as shown in Figs. 6 and 7.

Disengagement of the syringe is detected in the following manner. After the hooks 53 and 54 are opened upon operation of the clutch lever 52, when the user sets the flange of the thumb rest of the syringe inside the press surface of the cover 55, the contact pin 1 becomes flush with the press surface of the thumb rest. In cooperation with the motion of the contact pin 1, the contact pin guide 5 moves in the right-to-left direction as shown in Fig. 5. The contact pin guide 5 also pivots the dog portion 9b, and this pivot motion is detected by the photosensor 46 serving as a detecting means. If the angle of pivot motion does not fall within a predetermined range (0° to 4°), it is determined that the syringe is not mounted.

In other words, even after the syringe pump is started, presence/absence of the flange of the thumb rest can be monitored by the photosensor 46.

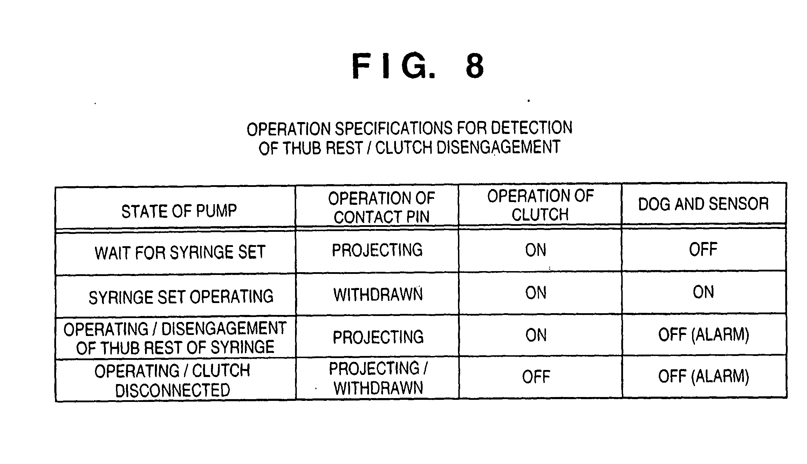

With the above arrangement, disengagement of the flange of the thumb rest of the syringe during operation can be detected, as shown by the operation specification table of Fig. 8. Also, an alarm is produced, if necessary, to warn the user.

The syringe pump described above allows detection of whether the flange of the thumb rest of the syringe is set on the slider assembly of the syringe pump, regardless of whether the pump is in operation or stopped and the flange of the thumb rest can be reliably fixed to the slider assembly, and which is not affected by electric noise.

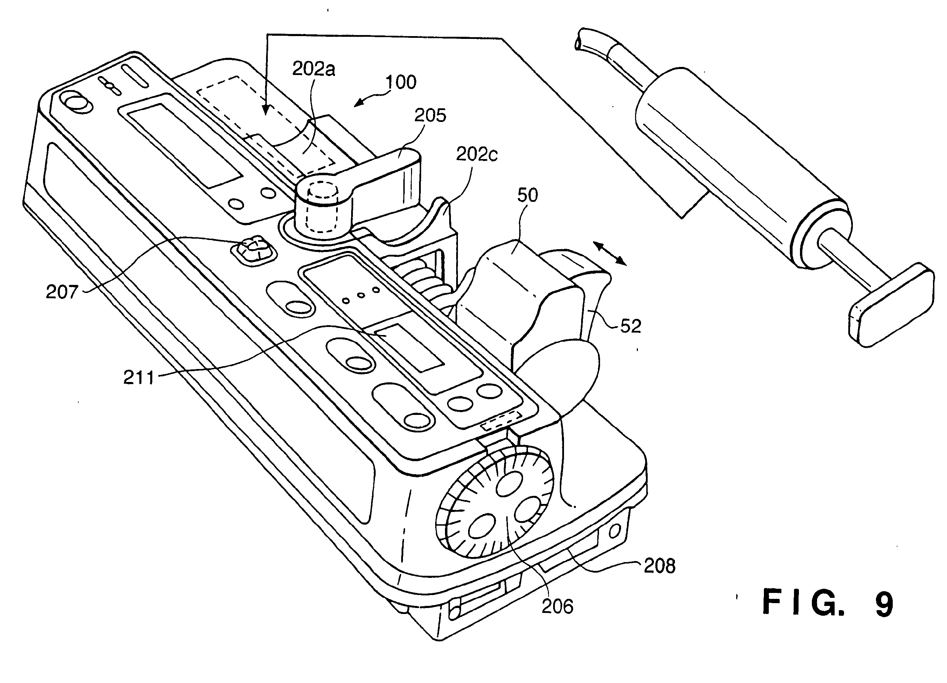

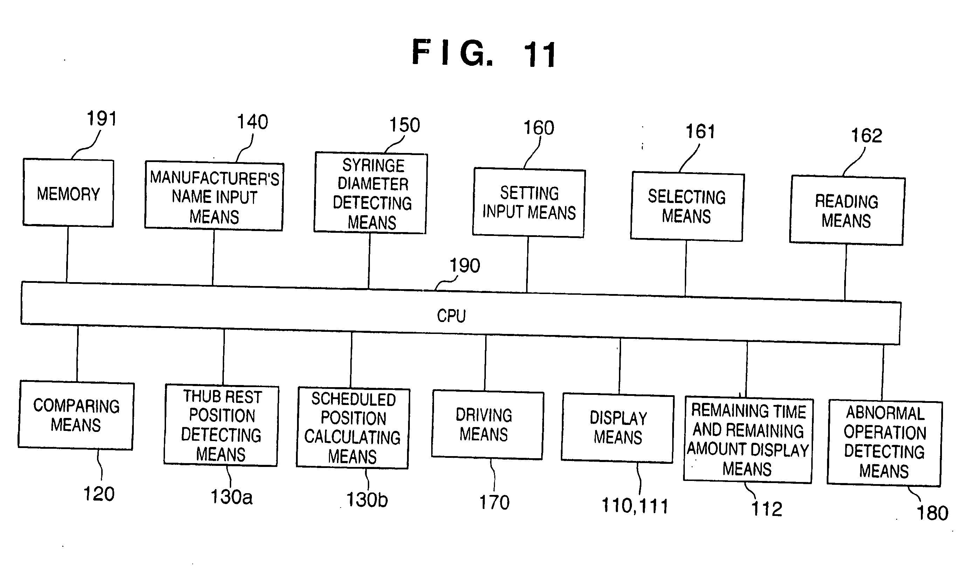

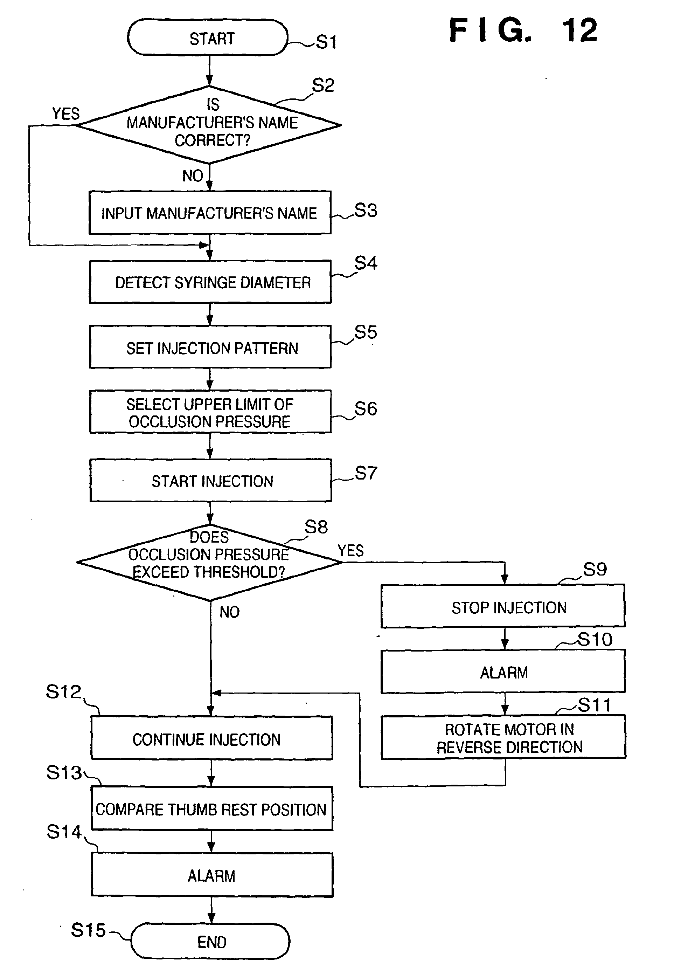

How to use an example of the arrangement of the syringe pump which uses the mechanism of the syringe pump described above will be described with reference to the accompanying drawings. Fig. 9 is a perspective view of the outer appearance of the syringe pump, Fig. 10 is a plan view of an operation panel, Fig. 11 is a block diagram of the syringe pump, and Fig. 12 is a flow chart for explaining the operation of a syringe pump according to the present invention.

As a preparation, the instruments are checked, and whether a syringe pump 100, a pole clamp attached to the syringe pump 100, an AC power cable, a transfusion stand (not shown), a syringe (not shown) containing a liquid drug, and an indwelling needle are all prepared is checked. After that, the pole clamp (not shown) is firmly fixed to the transfusion stand. For this purpose, a pole clamp attaching screw is inserted in a screw hole in the bottom surface of the syringe pump 100, and is set by screwing. The AC power cable is connected to an AC inlet 208 at the right surface of the main body, and the plug is connected to an AC 100 V outlet with a ground terminal.

When the AC power is connected, a battery lamp 217 is turned on to indicate that a built-in battery is being charged.

When the user presses a power switch 215 for about 1 sec to turn on the power supply, all lamps flash three times, and a buzzer sounds to perform self check automatically (step S1). An AC/DC lamp 216 is turned on, and flow rate, scheduled amount, and integrated amount display lamps 223, 224, and 225 are turned on. A flow rate, scheduled amount, and integrated amount display 211 displays a preset syringe manufacturer's name for about 3 sec in figures. The user must necessarily check whether the manufacturer of the set syringe coincides with the preset manufacturer (step S2).

If the syringe manufacturer is not correct, a correct manufacturer's name is set and input from a manufacture's name input unit (input means) 140 shown in the block diagram of Fig. 11 (step S3). The set and input manufacturer's name is displayed on the display 211 in characters or figures.

With a lapse of a predetermined period of time, the figures on the flow rate, scheduled amount, and integrated amount display 211 indicating the manufacturer's name disappear, and "0. 0" is displayed on the display 211. An operation indicator 207 is kept off.

A syringe type display lamp 218 displays by flashing all of its four built-in display lamps that a syringe (not shown) is not mounted, to prompt the user to set a syringe. The user sets syringe after all of the above display contents are checked.

An injection line (tube) and a syringe filled with a liquid drug are connected in a sterile atmosphere, and the main body of the syringe is set on a stage 202a shown in Fig. 9 and is locked with a clamp 205.

The user places the flange (not shown) of the syringe in a slit 202c in order to set it there. After that, the user sets the main body of the syringe on the syringe stage 202a, and pivots the clamp 205 in a predetermined direction. Then, the syringe is unlocked, and is clamped. The syringe diameter (syringe volume) is detected by a syringe diameter detecting means (detector) 150 (step S4).

The user presses a clutch lever 52 of a slider assembly 50 to unlock the clutch, and moves the slider in the directions of arrows. When the clutch lever 52 of the slider assembly 50 is pressed, the slider can be moved manually.

After the thumb rest of the syringe abuts against the slider assembly 50, when the user releases the clutch lever 52, the left and right hooks 53 and 54 automatically hold the thumb rest. In other words, when the user releases the clutch lever 52, the hooks 53 and 54 of the slider assembly 50 sandwich the thumb rest of the syringe.

When the syringe is set, priming is performed. Priming must be necessarily performed before centesis to the patient. When the user presses a fast-forward switch 230, the operation indicator 207 performs rotational display. The pump starts operation and the liquid drug appears at the distal end of the indwelling needle. When the user keeps pressing the fast-forward switch 230 to supply the liquid drug to the distal end of the indwelling needle, the integrated amount lamp 225 flashes.

During fast-forwarding, the integrated amount lamp 225 flashes, and the flow rate, scheduled amount, and integrated amount display 211 displays a priming amount. The priming amount is added to the integrated amount in units of 0.1 mℓ . If the user presses an integration clear switch 227, the integrated amount can be cleared to "zero".

This priming is significant for eliminating the gap between the syringe and the main body so the acting surface of the slider assembly 50 of the main body abuts against the thumb rest of the syringe without any gap, and must accordingly be performed necessarily.

When priming described above is ended, an injection pattern (injection amount) is set at a setting input means (input unit) 160. Prior to setting, whether the flow rate lamp 223 is turned on is checked. If the flow rate lamp 223 is not turned on, the user presses a display selector switch 226 to turn on the flow rate lamp 223.

After that, a setting dial 206 forming part of the input unit 160 is dialed to set a flow rate per hour. In order to prevent erroneous operation of dial setting and to assure safety, the value does not change for a half revolution after the start of dialing. When the setting dial 206 is dialed over a half revolution, the buzzer sounds and the value changes.

The value decreases when the setting dial 206 is dialed counterclockwise, and increases when it is dialed clockwise. While pressing a stop/mute switch 228, when the user dials the setting dial 206 a value at an upper digit position changes rapidly.

Regarding the syringe type and the maximum flow rate, the maximum flow rate that can be set is determined in accordance with the type of the syringe. For example, for a 30 mℓ syringe, the maximum flow rate is 300 mℓ/h. When a value larger than the maximum flow rate is set and the start switch 229 is pressed, the flow rate preset value lamp flashes, and injection is not started. Therefore, the injection pattern is set again, and setting operation is completed (step S5).

The occlusion detection pressure level and the buzzer volume level are set by pressing the stop/mute switch 228 and display selector switch 226. To set or change the occlusion detection pressure level, while pressing the stop/mute switch 228, the user presses the display selector switch 226 simultaneously. Then, the flow rate, scheduled amount, and integrated amount display 211 displays "P***", and the setting mode is set. While keeping pressing the stop/mute switch 228, the user releases and presses the display selector switch 226. Then, characters "L" (low), "M" (medium), and "H" (high) printed near occlusion pressure set value lamps 219a, 219b, and 219c are turned on in this order. The user selects a desired occlusion pressure level, releases all the switches, and presses the stop/mute switch 228, and then the integration clear switch 227, thereby selecting, setting, and inputting an occlusion detection pressure level (step S6). "***" mentioned above corresponds to "L, M, and H" described above. In this manner, the stop/mute switch 228, display selector switch 226, and integration clear switch 227 serve as an occlusion pressure selecting means.

On the basis of the selected, set, and input occlusion pressure level, syringe diameter (volume), and syringe manufacturer's name, a corresponding one of occlusion pressure thresholds (upper limits and/or lower limits) which are made up into a table in advance is selected automatically, and injection of the liquid drug is started (step S7).

A predetermined number of data are sampled during a predetermined sampling period (e.g., 16 data are continuously sampled each every 0. 05 sec), the moving average of the data is calculated, and whether the occlusion pressure exceeds the threshold is checked (step S8).

If the occlusion pressure exceeds the threshold, injecting operation is stopped (step S9), and an alarm is produced and displayed (step S10). The motor is rotated for a predetermined amount to return the slider by an amount corresponding to a bolus amount ($V_0(m\ell)$) unique to a predetermined stored cylinder (the motor is rotated in the reverse direction by a predetermined amount), or until reaching a value corresponding to a venous pressure ($F_{iv}(kgf)$) (step S11).

If the occlusion pressure does not exceed the threshold, injection is continued (step S12). On the basis of data selected and input by a remaining amount/remaining time selection switch (selecting means), a pre-alarm position LNE (cm) is calculated by a controller (CPU) 190 in the following manner.

<Setting with Remaining Amount>

Assume that the pre-alarm position LNE is to be set with a preset remaining amount VNE (m$\ell$). On the basis of the stored data of the table described above, an alarm position LNE (cm) is calculated as:

$$\texttt{thumb rest push end position LNE (cm)}$$
$$\texttt{= LE (cm) + VNE (m}\ell\texttt{)/A (cm}^2\texttt{)}$$

where LE (cm) is the thumb rest push end position and A (cm$^2$) is the syringe sectional area.

<Setting with Remaining Time>

Assume that the pre-alarm position LNE is to be set with the preset remaining amount VNE (m$\ell$). On the basis of the stored data of the table described above, the alarm position LNE (cm) is calculated as:

$$\texttt{thumb rest push end position LNE (cm)}$$
$$\texttt{= LE (cm) + R (m}\ell\texttt{/h) x T (h)/A (cm}^2\texttt{)}$$

where R (m$\ell$/h) is the injection rate, T (h) is the remaining time, and A (cm$^2$) is the syringe sectional area.

Comparative calculation of the pre-alarm position of the thumb rest is performed in the above manner (step S13). When the thumb rest reaches the pre-alarm position, an alarm is produced, and the operation indicator 207 is turned on to emit yellow light rotationally (flashes) (step S14). With a lapse of a predetermined period of time, injection is ended (step S15).

After the syringe is set on the syringe pump 100, if injection is not started with a lapse of about 2 min since flow rate setting operation, the buzzer sounds to inform the user that he might forget to start the operation. When the user presses the stop/mute switch 228 in response to the buzzer, the buzzer stops. If the user wishes to change the injection pattern (flow rate) during injection, he performs temporary stop of injection to stop injection, and sets the injection pattern again with the setting input means 160 including the setting dial 206. At this time, when the user presses the stop/mute switch 228, the operation indicator 207 is turned off.

When a scheduled amount of liquid drug is injected, the user checks the stop state and removes the syringe. For this purpose, the user pulls up the clamp 205, rotates it through about 90°, and holds it in this state. The user then presses the clutch lever 52 of the slider assembly 50 to open left and right hooks 53 and 54, and removes the syringe. After that, if the user will not use the syringe pump again, he presses the power switch 215 for about 2 sec or more to turn it off.

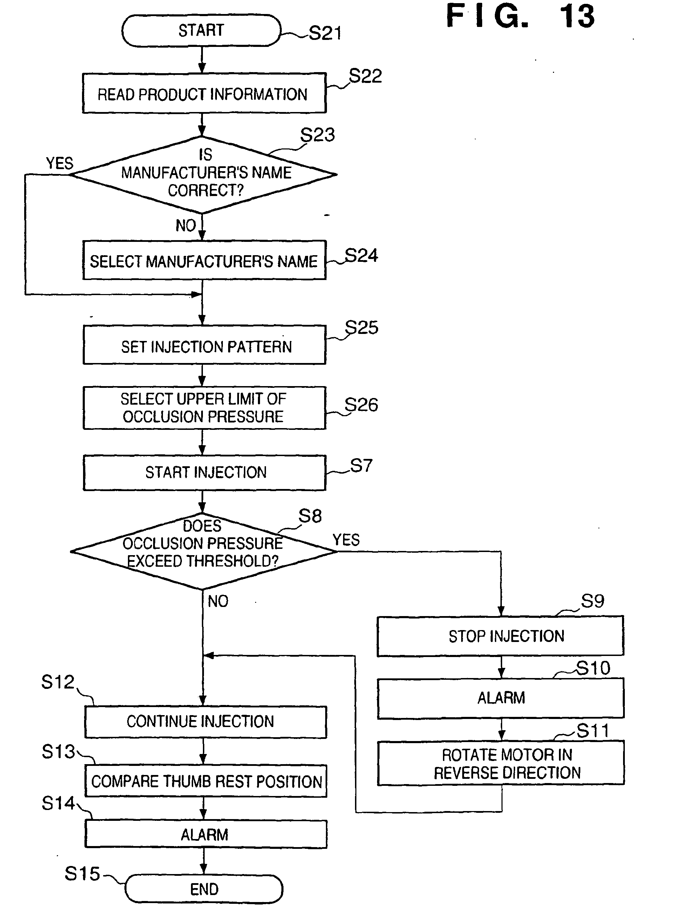

A case wherein product information adhered to the syringe is to be automatically read by a reading means 162 will be described with reference to the operation flow chart of Fig. 13 and the block diagram of Fig. 11.

When the user presses the power switch 215 for about 1 sec to turn on the power supply, all lamps flash three times, and a buzzer sounds to perform self check automatically (step S21). The AC/DC lamp 216 is turned on, and the flow rate, scheduled amount, and integrated amount display lamps 223, 224, and 225 are turned on. The flow rate, scheduled amount, and integrated amount display 211 displays a preset syringe manufacturer's name for about 3 sec in figures. The product information (bar code) of the set syringe is automatically read by the product information reading means 162 to identify the type of syringe (step S22). The user must necessarily check whether the manufacturer of the set syringe coincides with the preset manufacturer (step S23).

If the syringe manufacturer is different from the preset one, a correct manufacturer's name is set and input from the manufacture's name input unit (input means) 140 (step S24). If the syringe manufacturer is identical to the preset one, the next injection pattern is set (step S25). The set and input manufacturer's name is displayed on the display 211 in characters or figures.

When the injection pattern is set and input, an occlusion pressure threshold is set (step S26). How to use the syringe pump after this is identical to that of the operation flow chart of Fig. 12, and a detailed description thereof will be omitted.

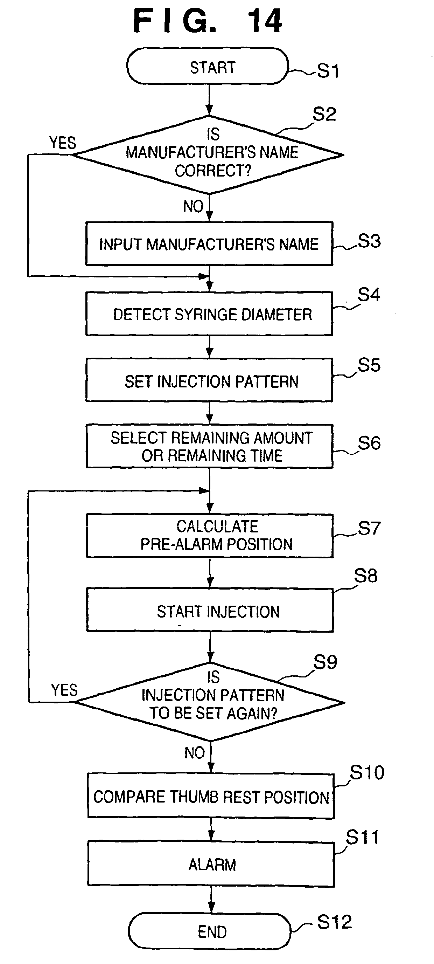

Fig. 14 is a flow chart for explaining the operation of a comparative syringe pump. In Fig. 14, steps that are identical to those already described with reference to the operation flow chart of Fig. 12 will be denoted by the same reference numerals as in Fig. 12, and a detailed description thereof will be omitted.

When the injection pattern is set in step S5, the flow advances to step S6, and the user selects and inputs whether an alarm is to be produced when the liquid drug in the syringe has reached a predetermined remaining amount or when the thumb rest of the syringe has reached a predetermined position with the remaining amount/remaining time selection switch of a selecting means 161. On the basis of the selected input data and the preset input value of the injection pattern of the flow rate, the pre-alarm position LNE (cm) is calculated by a controller 190 in the following manner (step S7).

<Setting with Remaining Amount>

Assume that the pre-alarm position LNE is to be set with a preset remaining amount VNE (m$\ell$). On the basis of the stored data of the table described above, an alarm position LNE (cm) is calculated as: thumb rest push end position LNE (cm) = LE (cm) + VNE (m$\ell$) /A (cm$^2$) where LE (cm) is the thumb rest push end position.

<Setting with Remaining Time>

Assume the pre-alarm position LNE is to be set with a preset remaining amount VNE (m$\ell$). On the basis of the stored data of the table described above, the alarm position LNE (cm) is calculated as: thumb rest push end position LNE (cm) = LE (cm) + R (m$\ell$/h) x T (h)/A (cm$^2$) where R (m$\ell$/h) is the injection speed, T (h) is the remaining time, and A (cm$^2$) is the syringe sectional area.

When the injection pattern is set in the above manner, the user checks whether the main body 1 is in the stop state, and punctures the patient' s arm or the like with an indwelling needle. When all the operations are completed, the liquid drug is injected. When the user presses a start switch 229 for this purpose, injection is started (step S8). Since an operation indicator 207 is turned on rotationally, the user can check the injection state of the liquid drug at a place far from the syringe pump.

After the syringe is set on a main body 100, if injection is not started with a lapse of about 2 min since flow rate setting operation, the buzzer sounds to inform the user that he might forget to start the operation. When the user presses a stop/mute switch 228 in response to the buzzer, the buzzer stops. If the user wishes to change the flow rate injection pattern during injection, he performs temporary stop of injection to stop injection, and sets the injection pattern again with a setting input means 160 including a setting dial 206 (step S9). At this time, when the user presses the stop/mute switch 228, the operation indicator 207 is turned off.

After injection is started, the user checks the integrated amount. For this purpose, the user presses a display selector switch 226 to turn on an integration amount lamp 225. Then, a flow rate, scheduled amount, and integrated amount display 211 displays an integrated amount since the start of injection for about 15 sec in units of 0.1 m$\ell$.

To clear the integrated amount, the user presses the stop/mute switch 228 to stop the main body 100, the display selector switch 226 to display the integrated amount, and then an integration clear switch 227 for about 1.5 sec. The buzzer sounds, and the integrated amount of the flow rate, scheduled amount, and integrated amount display 211 is cleared to "0.01 mℓ". A thumb rest position detected by a thumb rest position detecting means (detection unit) 130a including a potentiometer is compared with the pre-alarm position by a comparing means 120 (step S10). If it is determined that the thumb rest has reached the pre-alarm position, the operation indicator 207 makes an alarm (step S11).

When a scheduled amount of liquid drug is injected, the user removes the syringe in step S12. For this purpose, the user pulls up the clamp 205, rotates it through about 90°, and holds it in this state. The user then presses a clutch lever 52 of a slider assembly 50 to open left and right hooks 53 and 54, and removes the syringe. After that, if the user will not use the syringe pump again, he presses a power switch 215 for about 2 sec or more to turn it off.

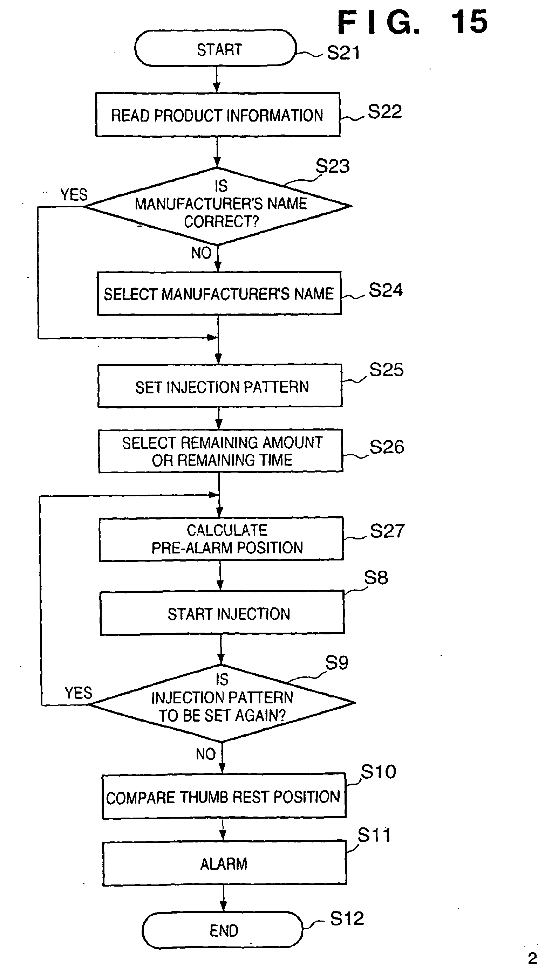

A case wherein product information adhered to the syringe is to be automatically read by a reading means 162 will be described with reference to the operation flow chart of Fig. 15 and the block diagram of Fig. 11.

When the user presses the power switch 215 for about 1 sec to turn on the power supply, all lamps flash three times, and a buzzer sounds again to perform self check automatically (step S21). An AC/DC lamp 216 is turned on, and flow rate and scheduled amount display lamps 223 and 224 and the integrated amount display lamp 225 are turned on. The flow rate, scheduled amount, and integrated amount display 211 displays a preset syringe manufacturer's name for about 3 sec in figures. The product information (such as bar code) of the set syringe is automatically read by the product information reading means 162 to specify the type of syringe (step S22). The user must necessarily check whether the manufacturer of the set syringe coincides with the preset manufacturer's name (step S23).

If the syringe manufacturer is different from the preset one, a correct manufacturer's name is set and input from a manufacture's name input unit (input means) 140 (step S24). If the syringe manufacturer is identical to the preset one, the next injection pattern is set (step S25). The set and input manufacturer's name is displayed on the display 211 in characters or figures. If the syringe manufacturer's name is correct, the injection pattern is set and input in step S25.

When the injection pattern is set and input, the user selects and inputs whether an alarm is to be produced when the liquid drug in the syringe has reached a predetermined remaining amount or when the thumb rest of the syringe has reached a predetermined position with the remaining amount and remaining time selection switch of the selecting means 161 (step S26).

<Setting with Remaining Amount>

Assume that the pre-alarm position LNE is to be set with a preset remaining amount VNE (mℓ). On the basis of the stored data of the table described above, an alarm position LNE (cm) is calculated as:

$$\texttt{thumb rest push end position LNE (cm)}$$
$$\texttt{= LE (cm) + VNE (m}\ell\texttt{)/A (cm}^2\texttt{)}$$

where LE (cm) is the thumb rest push end position and A (cm$^2$) is the syringe sectional area.

<Setting with Remaining Time>

Assume that the pre-alarm position LNE is to be set with a preset remaining amount VNE (mℓ). On the basis of the stored data of the table described above, the alarm position LNE (cm) is calculated as:

$$\texttt{thumb rest push end position LNE (cm)}$$
$$\texttt{= LE (cm) + R (m}\ell\texttt{/h) x T (h)/A (cm}^2\texttt{)}$$

where R (mℓ/h) is the injection speed, T (h) is the remaining time, and A (cm$^2$) is the syringe sectional area.

The flow then advances to step S27 corresponding to step S7 of Fig. 14, steps S8, S9, S10, S11, and S12 similar to those Fig. 14 are performed, and the flow is ended.

The syringe pump with the above functions can sufficiently achieve desired functions. More effective liquid drug injection can be performed with an injection setting function per weight, e.g., μg/kg/min, mg/kg/h, or the like.

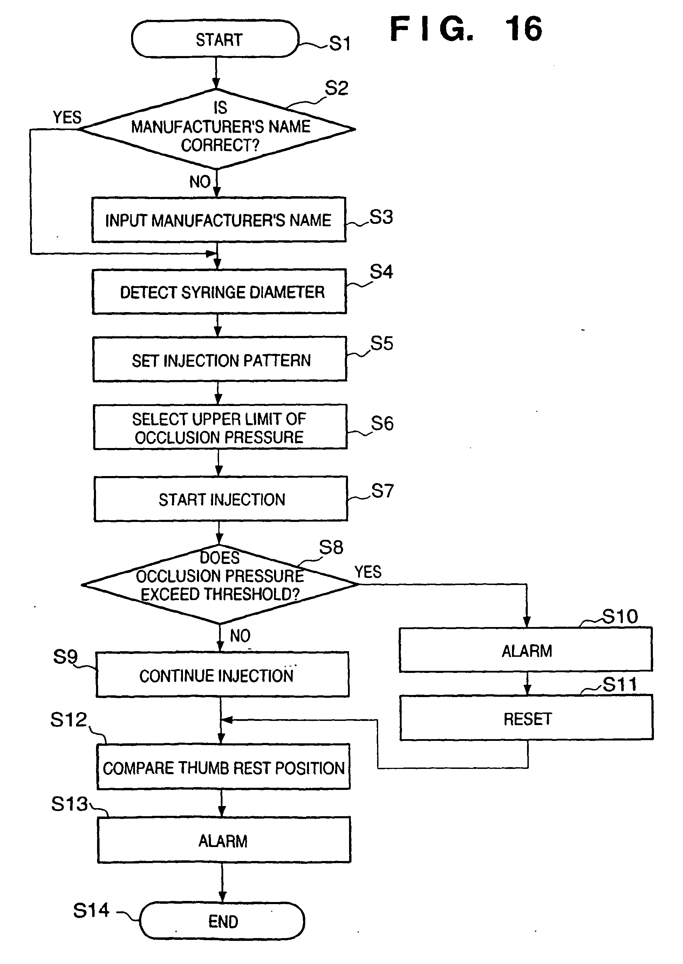

Fig. 16 is a flow chart for explaining the operation of a comparative syringe pump In Fig. 16, steps that are identical to those already described with reference to the operation flow chart of Fig. 12 will be denoted by the same reference numerals as in Fig. 12, and a detailed description thereof will be omitted.

In Fig. 16, steps S1 to S7 are identical to those of the operation flow chart of Fig. 12.

In step S8, a predetermined number of data are sampled during a predetermined sampling period (e.g., 16 data are continuously sampled each every 0.05 sec), the moving average of the data is calculated, and whether the occlusion pressure exceeds the threshold is checked (step S8).

If the occlusion pressure exceeds the threshold, an alarm is produced, and an operation indicator 207 is turned on or flashes in red to inform the alarm to the user (step S10). The user checks the occlusion pressure. If there is no problem, the syringe pump is reset (step S11).

Steps S12, S13, and S14 are performed, and the flow is ended.

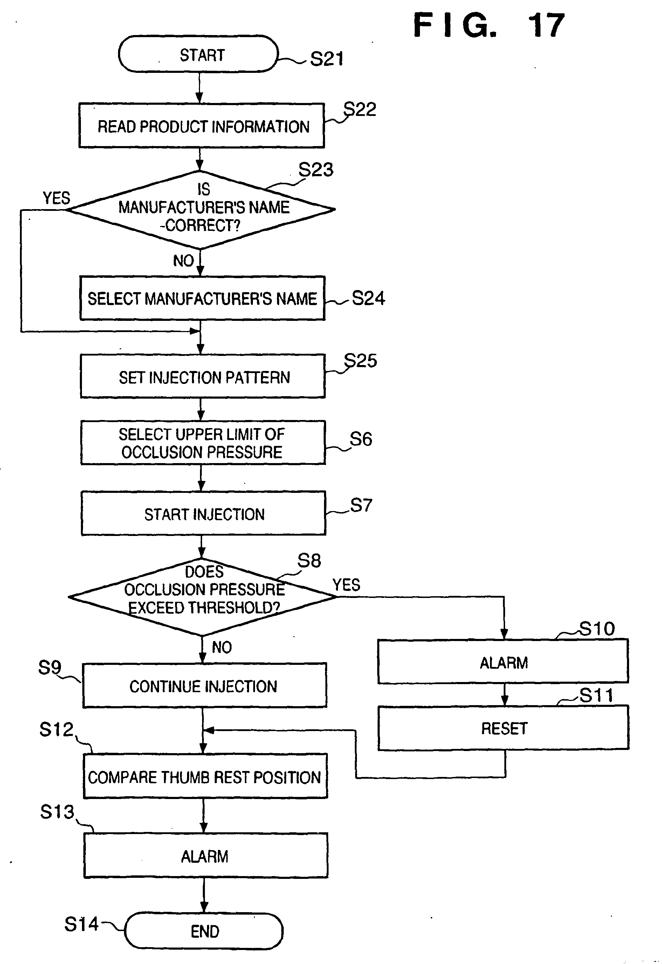

Finally, a case wherein product information adhered to the syringe is to be automatically read by a reading means 162 will be described with reference to the operation flow chart of Fig. 17 and the block diagram of Fig. 11.

When the user presses a power switch 215 for about 1 sec to turn on the power supply, all lamps flash three times, and a buzzer sounds again to perform self check automatically (step S21). An AC/DC lamp 216 is turned on, and flow rate, scheduled amount, and integrated amount display lamps 223, 224, and 225 are turned on. A flow rate, scheduled amount, and integrated amount display 211 displays a preset syringe manufacturer's name for about 3 sec in figures. The product information (bar code) of the set syringe is automatically read by the product information reading means 162 to identify the type of syringe (step S22). The user must necessarily check whether the manufacturer of the set syringe coincides with the preset-manufacturer (step S23).

If the syringe manufacturer is different from the preset one, a correct manufacturer's name is set and input from a manufacture's name input unit (input means) 140 (step S24).If the syringe manufacturer is identical to the preset one, the next injection pattern is set (step S25). The set and input manufacturer's name is displayed on the display 211 in characters or figures.

When the injection pattern is set and input, an occlusion pressure threshold is set (step S26). How to use the syringe pump after this is identical to that of the operation flow chart of Fig. 16, and a detailed description thereof will be omitted.

With the syringe pump described above, occlusion detection can be performed more accurately with various types of injection patterns (program input patterns) and various types of syringes, and drive control of the syringe can be performed without causing bolus, even when an occlusion is detected.

An alarm can be produced a predetermined period of time (hours, minutes) before scheduled injection end time, based on a predetermined liquid drug remaining amount in the syringe and/or on a predetermined position of the thumb reset of the syringe before liquid drug injection is ended, and not on the various types of injection patterns (program input patterns). A syringe pump can be obtained which can arbitrarily select an occlusion pressure for various types of syringes in accordance with syringe volumes (syringe diameters) and syringe manufacturer or for a syringe containing a predetermined liquid drug, so that it can detect an occlusion pressure precisely.

**Claims**

1.  A syringe pump for injecting a liquid drug in a syringe in accordance with a predetermined injection pattern, comprising:

    either:

    syringe diameter detecting means (150) for detecting a volume of the syringe, and
    manufacturer setting means (140) for setting and inputting a manufacturer of the syringe,

    or:

    reading means (162) for reading identification information attached to a set syringe to specify a type of the syringe including a manufacturer of the syringe and a volume of the syringe, and

    said syringe pump further comprising:

    injection pattern input means (160) for inputting an injection pattern,
    storage means (191) for storing data including a sectional area of the syringe, a slide resistance of the syringe, an outer diameter of the syringe, a predetermined slider return amount on detection of occlusion when the syringe is being operated to inject, and an upper limit of an occlusion pressure of the syringe in the form of a table in units of at least one syringe manufacturer and syringe volume,
    discriminating means (150, 190) for specifying the set syringe on the basis of the detected outer diameter of the syringe and the set and input name of the syringe manufacturer,
    determining means (190) for determining a threshold of the occlusion pressure on the basis of information selected from the specified syringe and the upper limit of the occlusion pressure, and
    comparing means (120) for comparing the detected occlusion pressure of the syringe with the threshold,

the syringe further having control means arranged to perform a control operation on the basis of a result of said comparing means (120) and in accordance with the specified syringe, to return said slider by the predetermined slider return amount, said amount corresponding to a predetermined volume, or until a load acting on said slider becomes not more than a predetermined value.

**Patentansprüche**

1. Eine Spritzenpumpe zum Einspritzen eines flüssigen Arzneimittels in einer Spritze entsprechend einem vorbestimmten Einspritzmuster, die aufweist:

entweder eine Spritzendurchmesser-Erfassungseinrichtung (150) zum Erfassen eines Volumens der Spritze und
eine Hersteller-Einstelleinrichtung (140) zum Einstellen und Eingeben eines Herstellers der Spritze,
oder
eine Leseeinrichtung (162) zum Lesen von Identifikationsinformationen, die an einer eingesetzten Spritze befestigt sind, um einen Typ der Spritze einschließlich eines Herstellers der Spritze und ein Volumen der Spritze zu spezifizieren, und
wobei die Spritzenpumpe ferner aufweist:

eine Einspritzmuster-Eingabeeinrichtung (160) zum Eingeben eines Einspritzmusters,
eine Speichereinrichtung (191) zum Speichern von Daten einschließlich eines Schnittbereiches der Spritze, eine Gleitwiderstandes der Spritze, eines Außendurchmessers der Spritze, eines vorbestimmten Schieberrückführbetrages bei Erfassen einer Okklusion, wenn die Spritze zum Einspritzen betätigt wird, und eine obere Grenze eines Okklusionsdrucks der Spritze in Form einer Tabelle in Einheiten von zumindest einem Spritzenhersteller und Spritzenvolumen,
eine Unterscheidungseinrichtung (150, 190) zum Spezifizieren der eingesetzten Spritzen auf der Grundlage des erfassten Außendurchmessers der Spritze und des eingestellten und eingegebenen Namens des Spritzenherstellers,
eine Bestimmungseinrichtung (190) zum Bestimmen eines Schwellwertes des Okklusionsdrucks auf der Grundlage der Information, die von der spezifizierten Spritze ausgewählt wurde, und der obere Grenze des Okklusionsdrucks, und
eine Vergleichseinrichtung (120) zum Vergleichen des erfassten Okklusionsdrucks der Spritze mit dem Schwellwert,
wobei die Spritze ferner eine Steuereinrichtung hat, die angeordnet ist, um einen Steuervorgang auf der Grundlage eines Ergebnisses der Vergleichseinrichtung (120) und entsprechend der spezifizierten Spritze auszuführen, um den Schieber um den vorbestimmten Schieberrückführbetrag zurückzuführen, wobei der Betrag einem vorbestimmten Volumen entspricht, oder bis eine auf den Schieber wirkende Last nicht größer als ein vorbestimmter Wert wird.

**Revendications**

1. Pompe de seringue pour injecter un médicament liquide dans une seringue selon un modèle d'injection prédéterminé, comprenant :

soit :

des moyens de détection de diamètre de seringue (150) pour détecter un volume de la seringue, et
des moyens de réglage de fabricant (140) pour régler et entrer un fabricant de la seringue,

ou ben :

des moyens de lecture (162) pour lire l'information d'identification fixés à une seringue réglée afin de spécifier un type de la seringue comprenant un fabricant de la seringue et un volume de la seringue, et

ladite pompe de seringue comprenant en outre :

des moyens d'entrée de modèle d'injection (160) pour entrer un modèle d'injection,

des moyens de mémorisation (191) pour mémoriser des données comprenant une surface transversale de la seringue, une résistance au coulissement de la seringue, un diamètre externe de la seringue, une quantité de retour de coulisse prédéterminée suite à la détection d'occlusion lorsque la seringue est actionnée pour l'injection, et une limite supérieure d'une pression d'occlusion de la seringue sous la forme d'un tableau en unités d'au moins un fabricant de seringue et un volume de seringue,

des moyens de discrimination (150, 190) pour spécifier la seringue réglée en fonction du diamètre externe détecté de la seringue et le nom déterminé et entré du fabricant de la seringue,

des moyens de détermination (190) pour déterminer un seuil de la pression d'occlusion en fonction de l'information sélectionnée à partir de la seringue spécifiée et la limite supérieure de la pression d'occlusion, et

des moyens de comparaison (120) pour comparer la pression d'occlusion détectée de la seringue avec le seuil,

la seringue ayant en outre des moyens de commande agencés pour réaliser une opération de commande en fonction d'un résultat desdits moyens de comparaison (120) et selon la seringue spécifiée, pour faire revenir ladite coulisse par la quantité de retour de coulisse prédéterminée, ladite quantité correspondant à un volume prédéterminé, ou jusqu'à ce qu'une charge agissant sur ladite coulisse devienne non supérieure à une valeur prédéterminée.

# FIG. 1

# F I G. 2

# F I G. 3

# F I G. 4

# F I G. 5

# F I G. 6

# FIG. 7

# F I G. 8

OPERATION SPECIFICATIONS FOR DETECTION
OF THUB REST / CLUTCH DISENGAGEMENT

| STATE OF PUMP | OPERATION OF CONTACT PIN | OPERATION OF CLUTCH | DOG AND SENSOR |
|---|---|---|---|
| WAIT FOR SYRINGE SET | PROJECTING | ON | OFF |
| SYRINGE SET OPERATING | WITHDRAWN | ON | ON |
| OPERATING / DISENGAGEMENT OF THUB REST OF SYRINGE | PROJECTING | ON | OFF (ALARM) |
| OPERATING / CLUTCH DISCONNECTED | PROJECTING / WITHDRAWN | OFF | OFF (ALARM) |

EP 1 679 091 B1

F I G. 9

EP 1 679 091 B1

# FIG. 10

EP 1 679 091 B1

# FIG. 11

Block diagram:

- 191 — MEMORY
- 140 — MANUFACTURER'S NAME INPUT MEANS
- 150 — SYRINGE DIAMETER DETECTING MEANS
- 160 — SETTING INPUT MEANS
- 161 — SELECTING MEANS
- 162 — READING MEANS

- 190 — CPU

- 120 — COMPARING MEANS
- 130a — THUB REST POSITION DETECTING MEANS
- 130b — SCHEDULED POSITION CALCULATING MEANS
- 170 — DRIVING MEANS
- 110,111 — DISPLAY MEANS
- 112 — REMAINING TIME AND REMAINING AMOUNT DISPLAY MEANS
- 180 — ABNORMAL OPERATION DETECTING MEANS

EP 1 679 091 B1

**F I G. 12**

START — S1

IS MANUFACTURER'S NAME CORRECT? — S2

YES

NO

INPUT MANUFACTURER'S NAME — S3

DETECT SYRINGE DIAMETER — S4

SET INJECTION PATTERN — S5

SELECT UPPER LIMIT OF OCCLUSION PRESSURE — S6

START INJECTION — S7

DOES OCCLUSION PRESSURE EXCEED THRESHOLD? — S8

YES

NO

STOP INJECTION — S9

ALARM — S10

ROTATE MOTOR IN REVERSE DIRECTION — S11

S12 — CONTINUE INJECTION

S13 — COMPARE THUMB REST POSITION

S14 — ALARM

S15 — END

**F I G. 13**

START ～S21

READ PRODUCT INFORMATION ～S22

IS MANUFACTURER'S NAME CORRECT? ～S23

YES

NO

SELECT MANUFACTURER'S NAME ～S24

SET INJECTION PATTERN ～S25

SELECT UPPER LIMIT OF OCCLUSION PRESSURE ～S26

START INJECTION ～S7

DOES OCCLUSION PRESSURE EXCEED THRESHOLD? ～S8

YES

NO

STOP INJECTION ～S9

ALARM ～S10

ROTATE MOTOR IN REVERSE DIRECTION ～S11

S12 — CONTINUE INJECTION

S13 — COMPARE THUMB REST POSITION

S14 — ALARM

S15 — END

# F I G. 14

```
              START ───S1
                │
                ▼
YES         ┌─────────────┐ ───S2
┌───────────│     IS      │
│           │ MANUFACTURER'S NAME │
│           │   CORRECT?  │
│           └─────────────┘
│                │ NO
│                ▼
│    ┌──────────────────────┐ ───S3
│    │ INPUT MANUFACTURER'S NAME │
│    └──────────────────────┘
│                │
└────────────────┤
                 ▼
     ┌──────────────────────┐ ───S4
     │  DETECT SYRINGE DIAMETER  │
     └──────────────────────┘
                 │
                 ▼
     ┌──────────────────────┐ ───S5
     │   SET INJECTION PATTERN   │
     └──────────────────────┘
                 │
                 ▼
     ┌──────────────────────┐ ───S6
     │  SELECT REMAINING AMOUNT  │
     │    OR REMAINING TIME      │
     └──────────────────────┘
                 │
┌────────────────┤
│                ▼
│    ┌──────────────────────┐ ───S7
│    │       CALCULATE       │
│    │  PRE-ALARM POSITION   │
│    └──────────────────────┘
│                │
│                ▼
│    ┌──────────────────────┐ ───S8
│    │    START INJECTION    │
│    └──────────────────────┘
│                │
│                ▼
│ YES       ┌─────────────┐ ───S9
└───────────│     IS      │
            │ INJECTION PATTERN │
            │  TO BE SET AGAIN? │
            └─────────────┘
                 │ NO
                 ▼
     ┌──────────────────────┐ ───S10
     │ COMPARE THUMB REST POSITION │
     └──────────────────────┘
                 │
                 ▼
     ┌──────────────────────┐ ───S11
     │        ALARM          │
     └──────────────────────┘
                 │
                 ▼
               END ───S12
```

# FIG. 15

START ～S21

READ PRODUCT INFORMATION ～S22

IS MANUFACTURER'S NAME CORRECT? S23

YES

NO

SELECT MANUFACTURER'S NAME ～S24

SET INJECTION PATTERN ～S25

SELECT REMAINING AMOUNT OR REMAINING TIME ～S26

CALCULATE PRE-ALARM POSITION ～S27

START INJECTION S8

IS INJECTION PATTERN TO BE SET AGAIN? S9

YES

NO

COMPARE THUMB REST POSITION S10

ALARM S11

END S12

2

# FIG. 16

```
          ┌─────────────────┐
          │      START      │───S1
          └─────────────────┘
                   │
                   ▼
          ◇─────────────────◇ ──S2
  YES    ╱        IS         ╲
  ┌─────◇  MANUFACTURER'S     ◇
  │      ╲   NAME CORRECT?    ╱
  │       ◇─────────────────◇
  │                │ NO
  │                ▼
  │      ┌─────────────────────────┐
  │      │ INPUT MANUFACTURER'S NAME│──S3
  │      └─────────────────────────┘
  │                │
  └────────────────▶
                   ▼
          ┌─────────────────────────┐
          │  DETECT SYRINGE DIAMETER │──S4
          └─────────────────────────┘
                   │
                   ▼
          ┌─────────────────────────┐
          │   SET INJECTION PATTERN  │──S5
          └─────────────────────────┘
                   │
                   ▼
          ┌─────────────────────────┐
          │   SELECT UPPER LIMIT OF  │──S6
          │   OCCLUSION PRESSURE     │
          └─────────────────────────┘
                   │
                   ▼
          ┌─────────────────────────┐
          │     START INJECTION      │──S7
          └─────────────────────────┘
                   │
                   ▼
          ◇─────────────────◇ ──S8
         ╱      DOES          ╲    YES
        ◇ OCCLUSION PRESSURE    ◇──────────┐
         ╲ EXCEED THRESHOLD?    ╱          │
          ◇─────────────────◇             │
                   │ NO                    ▼
   S9              ▼              ┌────────────────┐ ──S10
  ┌─────────────────────────┐     │     ALARM      │
  │    CONTINUE INJECTION    │     └────────────────┘
  └─────────────────────────┘              │
                   │                        ▼  ──S11
   S12             │              ┌────────────────┐
  ┌─────────────────────────┐     │     RESET      │
  │ COMPARE THUMB REST POSITION│◀──└────────────────┘
  └─────────────────────────┘
                   │
   S13             ▼
  ┌─────────────────────────┐
  │          ALARM           │
  └─────────────────────────┘
                   │
   S14             ▼
          ┌─────────────────┐
          │       END       │
          └─────────────────┘
```

# F I G. 17

START — S21

READ PRODUCT INFORMATION — S22

IS MANUFACTURER'S NAME -CORRECT? — S23

YES

NO

SELECT MANUFACTURER'S NAME — S24

SET INJECTION PATTERN — S25

SELECT UPPER LIMIT OF OCCLUSION PRESSURE — S6

START INJECTION — S7

DOES OCCLUSION PRESSURE EXCEED THRESHOLD? — S8

YES

NO

ALARM — S10

RESET — S11

S9 — CONTINUE INJECTION

S12 — COMPARE THUMB REST POSITION

S13 — ALARM

S14 — END

# FIG. 18

# F I G. 19

**EP 1 679 091 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9122237 A
- EP 514907 B1
- US 5425716 A